# EUROPEAN PATENT SPECIFICATION

(11) **EP 2 739 258 B1**
(45) Date of publication and mention of the grant of the patent: **02.08.2017**
(21) Application number: 12745469.2
(22) Date of filing: 02.08.2012
(51) Int. Cl.: A61K 8/25, A61K 8/34, A61K 8/81, A61K 8/86, A61Q 11/00

(54) **NON-AQUEOUS ORAL CARE COMPOSITIONS**
NICHTWÄSSRIGE MUNDPFLEGEMITTEL
COMPOSITIONS DE SOINS ORAUX NON AQUEUSES

(30) Priority: 03.08.2011 EP 11176458
(43) Date of publication of application: 11.06.2014
(73) Proprietor: Unilever PLC, London EC4Y 0DY (GB); Unilever N.V., 3013 AL Rotterdam (NL)
(72) Inventor: ASHCROFT, Alexander, Thomas, Bebington Wirral Merseyside CH63 3JW (GB); BRIGNOLI, Cinzia, I-26841 Casalpusterlengo (IT); CASERINI, Claudia, I-26841 Casalpusterlengo (IT); RAVIDA, Nunziatino, I-26841 Casalpusterlengo (IT); RUSTIONI, Andrea, I-26841 Casalpusterlengo (IT); SALTELLI, Roberta, I-26841 Casalpusterlengo (IT)
(74) Representative: Tansley, Sally Elizabeth
(86) International application number: PCT/EP2012/065163
(87) International publication number: WO 2013/017665

(56) References cited:
- WO-A1-01/45660
- WO-A1-96/03108
- DE-A1-102004 020 622
- US-A- 3 934 000
- US-A- 5 614 174
- Anonymous: "Zeodent High Performing Dental Silicas", Huber Dental Silica Products , September 2010 (2010-09), page 8PP, XP002667651, Retrieved from the Internet: URL:http://www.hubermaterials.com/userfile s/files/product-finder/saleslit/Zeodent%20 High%20Performing%20Dental%20Silicas.pdf [retrieved on 2012-01-19]

## Description

### Field of the Invention

The present invention is concerned with non-aqueous oral care compositions.

### Background of the Invention

Oral care compositions such as dentifrices typically contain dentally acceptable abrasive, humectant, water, and water-soluble polymer which serves as a t thickener and binder for the ingredients. A variety of other ingredients such as flavours, sweeteners, preservatives and fluoride are also utilized at low levels.

However there are many materials which are physically or chemically incompatible with the aqueous environments found in typical dentifrice formulations.

The need to stabilise both aqueous and anhydrous compositions, especially those compriding peroxides has been recognised by US 5 614 174 and WO 01/45660 respectfully.

Non-aqueous formulations have been suggested as a way of improving the stability of these materials. For example, WO96/03108 describes a non-aqueous dentifrice composition comprising a carboxyvinyl polymer, a humectant, a polyethylene glycol and a dentally acceptable abrasive. The carboxyvinyl polymer is stated to thicken the humectant materials and provide the necessary rheology in order to suspend any required abrasive material. A polyethylene glycol selected from PEG 300 and PEG 400 is stated to reduce stickiness from the formulation and give a smooth textured product.

A problem with non-aqueous formulations such as those disclosed in WO96/03108 is that they do not behave rheologically like a typical aqueous dentifrice. This problem is observed both during manufacture and during use by the consumer. It has led to manufacturing difficulties and reduced acceptance amongst consumers. Viscosity profile and flow characteristics are key factors governing ease of processing, product performance and consumer perception of a dentifrice.

The rheological behaviour of the above type of formulation may be improved by structuring the liquid continuous phase with a crystalline structuring agent, such as one or more solid polyethylene glycols having a melting point of 25°C or above. This leads to improved microstructure, ease of processing and sensory properties. However, the formulation may become cloudy and difficult to extrude, especially at high temperature. This may lead to an unattractive appearance and problems for the consumer in squeezing the formulation out of the tube in which it is packaged. The present inventors have found that this problem may be solved by the inclusion of certain silica particles.

### Summary of the Invention

The present invention provides a non-aqueous oral care composition with a liquid continuous phase comprising a thickening agent, a humectant, and a crystalline structuring agent, characterised in that the composition further comprises abrasive amorphous silica particles which are capable of acting as a booster to the cleaning ability of the composition and which have a weight mean particle size (d₅₀) ranging from 3 to 6 microns, as defined in claim 1. Surprisingly, the presence of the silica particles as defined above provides the composition with improved appearance (in particular shine or glossiness) and improved squeezability, even at high temperatures (such as 40°C)

### Detailed Description of the Invention

The composition of the invention is non-aqueous. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than about 5%, more preferably no greater than about 3% by weight based on the total weight of the composition.

### Silica Particles

The composition of the invention comprises abrasive amorphous silica particles which are capable of acting as a booster to the cleaning ability of the composition and which have a weight mean particle size (d₅₀) ranging from 3 to 6 microns.

The cleaning ability of the composition may be measured by assessing its effect on accumulated pellicle, for example by using the method developed by Stookey et al. (Journal of Dental Research 61 (1982), pp. 1236-1239). This model uses square bovine enamel blocks mounted in polymethylmethacrylate blocks. The specimens are polished and lightly acid etched in order to facilitate stain accumulation and adherence. They are attached to a staining apparatus which provides alternate immersion into a staining media and air drying at 37 °C. The staining media consists of tea, coffee, gastric mucin, sterilised trypticase soy broth and *Sarcina lutea* bacterial culture. After a number of days, the stained specimens are assessed by visual means using a five-point scale or the colour of the specimens may be measured objectively with a colorimeter. The stained specimens are then mounted in a mechanical brushing machine and the required load applied to each brush. The test toothpastes are dispersed in an aqueous diluent and the stained specimens brushed for a set number of brush strokes. The colour of the specimens is remeasured and from these values the amount of stain removed may be calculated against a reference standard (10 g of calcium pyrophosphate in 50 cm³ of 0.5% aqueous solution of sodium carboxymethyl cellulose). The Pellicle Cleaning Ratio (PCR) is calculated by means of the equation: [PCR =(% stain removed for Sample)/(% stain removed for Reference Standard) x 100]. The composition of the invention preferably has a PCR value of at least 30, more preferably at least 40, most preferably at least 50.

Preferred abrasive amorphous silica particles for use in the invention have Radioactive Dentine Abrasion Test (RDA) values ranging from 100 to 300, preferably 100 to 220. RDA values are unitless. A standard test procedure for measuring these values follows the method for assessment of dentifrice abrasivity recommended by the American Dental Association (Journal of Dental Research 55(4) 563, 1976). In this procedure, extracted human teeth are irradiated with a neutron flux and subjected to a standard brushing regime. The radioactive phosphorus 32 removed from the dentin in the roots is used as the index of the abrasion of the dentifrice tested. A reference slurry containing 10 g of calcium pyrophosphate in 50 cm³ of 0.5% aqueous solution of sodium carboxymethyl cellulose is also measured and the RDA of this mixture is arbitrarily taken as 100. In order to measure a powder RDA for the silica a suspension of 10.0 g of the silica in 50 cm³ of 0.5% aqueous solution of sodium carboxymethyl cellulose is prepared and the suspension is submitted to the same brushing regime.

Preferred abrasive amorphous silica particles for use in the invention have an oil absorption of 40 to 150 cm³ /100 g, and more preferably 40 to 100 cm³/100 g. A standard method for determining oil absorption is the ASTM spatula rub-out method (American Society of Test Material Standards D 281). The test is based on the principle of mixing linseed oil with the silica by rubbing with a spatula on a smooth surface until a stiff putty-like paste is formed which will not break or separate when it is cut with a spatula. The oil absorption is then calculated from the volume of oil (V cm³) used to achieve this condition and the weight, W, in grams, of silica by means of the equation: [oil absorption=(V*100)/W] (i.e. expressed in terms of cm³oil/100 g silica). The abrasive amorphous silica particles for use in the invention have a weight mean particle size in the range 3 to 6 microns. The weight mean particle size of the silica may suitably be determined using a Malvern Mastersizer® model S, with a 300 RF lens and MS 17 sample presentation unit. This instrument, made by Malvern Instruments, Malvern, Worcestershire, uses the principle of Fraunhofer diffraction, utilising a low power He/Ne laser. Before measurement, the sample is dispersed ultrasonically in water for 5 minutes to form an aqueous suspension. The Malvern Mastersizer® measures the weight particle size distribution of the silica. The weight mean particle size (d₅₀) or 50 percentile and the percentage of material below any specified size are easily obtained from the data generated by the instrument.

Preferably, the abrasive amorphous silica particles employed are precipitated silica.

Suitable precipitated silicas for use as abrasive amorphous silica particles in the invention are commercially available and include those marketed by PQ Corporation under the trade names SORBOSIL® AC 43 and SORBOSIL® AC 33. Mixtures of any of the above described materials may also be used.

The level of abrasive amorphous silica particles (as defined above) generally ranges from 0.05 to 10%, preferably from 0.1 to 5%, more preferably from 0.5 to 1%, by total weight abrasive amorphous silica particles (as defined above) based on the total weight of the composition.

### Thickening Agent

The liquid continuous phase of the composition of the invention comprises a thickening agent. The thickening agent helps to impart a desirable viscosity profile and desirable flow characteristics to the composition.

Suitable thickening agents for use in the invention are those which are operable in non-aqueous systems. Examples of such materials include organic macromolecules which do not necessarily require hydration with water in order to build viscosity, but can also build viscosity by alternative mechanisms such as by hydrogen bonding in the presence of hydroxyl donors such as polyols.

Carboxyvinyl polymers have been found to be useful in this context. The carboxyvinyl polymers for use in the invention have a molecular weight of at least about 750,000, more preferably at least about 1,250,000, most preferably at least about 3,000,000 g/mol. A suitable chemical class of such carboxyvinyl polymers for use in the invention includes crosslinked polymers having a polymer backbone derived from acrylic acid, substituted acrylic acid, or salts or esters thereof, in which the crosslinking agent contains two or more carbon-carbon double bonds and is derived from a polyhydric alcohol. Specific examples of such materials are homopolymers of acrylic acid cross-linked with allyl ethers of sucrose or pentaerythritol and homopolymers of acrylic acid cross-linked with divinyl glycol. An example of a suitable carboxyvinyl polymer for use in the compositions of the present invention is a homopolymer of acrylic acid crosslinked with allyl ethers of pentaerythritol, which is slightly pre-neutralised (1 to 3%) by potassium salt. This pre-neutralisation is done in order to precipitate polyacrylic acid in the presence of the polymerisation solvent. Such a material is commercially available, for example, as CARBOPOL® 974P NF Polymer, ex Lubrizol Advanced Materials, Inc. For ease of processing, the most preferred carboxyvinyl polymer for use in the compositions of the present invention is a homopolymer of acrylic acid crosslinked with pentaerythritol triallylether. Such a material is commercially available, for example, as SYNTHALEN® KP, ex 3V Sigma. Mixtures of any of the above described materials may also be used.

The level of thickening agent will depend on the particular type chosen, but generally ranges from 0.05 to 10% by total weight thickener based on the total weight of the composition. When the thickening agent is a carboxyvinyl polymer (as described above), the amount of carboxyvinyl polymer in compositions of the invention suitably ranges from 0.05 to 5%, preferably from 0.075 to 2%, more preferably from 0.05 to 0.5% by total weight carboxyvinyl polymer based on the total weight of the composition. For optimum performance (especially shine and squeezability) across a wide range of storage temperatures (e.g. 20 to 50°C), the amount of carboxyvinyl polymer ranges from 0.05 to 0.3% by total weight carboxyvinyl polymer based on the total weight of the composition.

### Humectant

The liquid continuous phase of the composition of the invention comprises a humectant. The humectant helps to keep the composition from hardening or crystallizing upon exposure to air. It also helps to give the composition a moist feel to the mouth, and may in some cases impart a desirable sweetness. The humectants for use in the invention are organic polyols having 3 or more hydroxyl groups in the molecule (hereinafter termed "organic polyols"). Examples of such materials include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The level of humectant will depend on the particular type chosen, but generally ranges from about 20 to 90% by weight based on the total weight of the composition. When the humectant is one or more organic polyols (as described above), the amount of organic polyol suitably ranges from 35 to 75%, more preferably from 45 to 70% by total weight organic polyol based on the total weight of the composition. Most preferably the composition of the invention is organic polyol-based. In the context of the present invention, the term "organic polyol-based" means that the composition is not oil-based or water-based, but instead, organic polyols (as defined above) are a principal component in the composition. By "principal component" is meant that the organic polyols (as defined above) when taken together, make up a higher portion of the composition's weight than any other compound. Ideally the composition of the invention is glycerol-based (i.e. glycerol makes up a higher portion of the composition's weight than any other compound) and contains from 45 to 70% by weight glycerol based on the total weight of the composition.

### Crystalline Structuring Agent

The liquid continuous phase of the composition of the invention comprises a crystalline structuring agent which is one or more solid polyethylene glycols having a melting point of from 35 to 65°C, more preferably from 55 to 60° C.

The crystals of the solid polyethylene glycol(s) help to provide an optimised microstructure for the composition.

Polyethylene glycols have the general formula H(OCH₂CH₂)ₙ OH, where n is the number of repeating oxyethylene units. Commercially available polyethylene glycols are usually not uniform chemical compounds, but instead consist of a distribution of similar polymer members of the homologous polyethylene glycol series, defined by average values of n and molecular weight. The melting point generally increases with increasing average values of n and molecular weight. Suitable solid polyethylene glycols have an average value of n in the above general formula ranging from about 20 to 220, preferably from about 40 to 150, more preferably from about 32 to 90, most preferably from about 60 to 75. The average molecular weight suitably ranges from about 950 to 11,250, preferably from about 1800 to 6600, more preferably from about 1400 to 4400, most preferably from about 2700 to 3700 g/mol. Suitable commercially available materials include for example Polyglykol® 3000 (ex Clariant). Mixtures of any of the above described materials may also be used.

The amount of solid polyethylene glycol (as defined above) in compositions of the invention suitably ranges from 0.1 to 5%, preferably from 0.5 to 3%, more preferably from 1 to 2.5% by total weight solid polyethylene glycol (as defined above) based on the total weight of the composition.

### Product Form and Optional Ingredients

The composition of the invention is typically in the form of a dentifrice. The term "dentifrice" denotes a formulation which are used to clean the surfaces of the oral cavity. The dentifrice is an oral composition that is not intentionally swallowed for purposes of systemic administration of therapeutic agents, but is retained in the oral cavity for a sufficient time to contact substantially all of the dental surfaces and/or mucosal tissues for purposes of oral activity. Preferably the dentifrice is suitable for application with a toothbrush and is rinsed off after use. Preferably the dentifrice is in the form of an extrudable semi-solid such as a cream, paste or gel (or mixture thereof).

A dentifrice composition according to the invention will generally contain further ingredients to enhance performance and/or consumer acceptability.

For example, the dentifrice may comprise other abrasive materials (in addition to the abrasive amorphous silica particles described above). Such other abrasive materials will generally be present in an amount of from 3 to 75% by weight based on the total weight of the dentifrice. Suitable other abrasive materials include other abrasive silicas (i.e. abrasive silicas which are different to the abrasive amorphous silica particles described above), calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Furthermore, a dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Preferably, the liquid continuous phase of the composition of the invention comprises one or more liquid polyethylene glycols having a melting point below 25°C. Preferably the melting point ranges from -50 to 22°C, more preferably from - 15 to 8° C, most preferably from 4 to 8°C.

The liquid polyethylene glycol(s) helps to make processing of the composition easier, especially at high shear, by reducing the overall viscosity of the liquid continuous phase.

Preferred liquid polyethylene glycols have an average value of n in the general formula H(OCH₂CH₂)ₙOH (as described above) ranging from about 4 to 12, more preferably about 6 to 8, most preferably about 8. The average molecular weight suitably ranges from about 190 to 630, more preferably from about 285 to 420, most preferably from about 380 to 420 g/mol. Suitable commercially available materials include for example PEG 400 (ex BDH Chemicals). Mixtures of any of the above described materials may also be used.

The amount of liquid polyethylene glycol (as defined above) in compositions of the invention suitably ranges from 1 to 20%, preferably from 5 to 15%, more preferably from 8 to 12% by total weight liquid polyethylene glycol (as defined above) based on the total weight of the composition.

Being non-aqueous, the composition of the invention is particularly suitable as a vehicle for oral care actives which may be physically or chemically incompatible with water, or which may function less efficiently in an aqueous environment.

Specific examples of oral care actives which may be included in the compositions of the invention include:
fluoride sources such as sodium fluoride, stannous fluoride, sodium monofluorophosphate, zinc ammonium fluoride, tin ammonium fluoride, calcium fluoride, cobalt ammonium fluoride and mixtures thereof;
plant-derivable antioxidants such as flavonoid, catechin, polyphenol, and tannin compounds and mixtures thereof;
antioxidant vitamins such as tocopherols and/or derivatives thereof, ascorbic acid and/or derivatives thereof and mixtures thereof.

Preferred examples of oral care actives for inclusion in the compositions of the invention include agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

Mixtures of any of the above described materials may also be used.

Compositions of the present invention may also contain further optional ingredients customary in the art such as anticaries agents, anticalculus agents, buffers, flavouring agents, sweetening agents, colouring agents, opacifying agents, preservatives, antisensitivity agents, antimicrobial agents and the like.

### Process

The invention also provides a process of preparing a non-aqueous oral care composition as defined above, comprising the steps of forming a mixture comprising the humectant and the solid polyethylene glycol(s), heating the mixture to a temperature above the melting point of the solid polyethylene glycol(s), and cooling the mixture to form crystals of the solid polyethylene glycol(s).

A preferred process according to the invention comprises the following steps:
(i) forming a mixture of humectant (e.g. glycerol) and liquid polyethylene glycol (e.g. PEG400),
(ii) heating the mixture to a temperature above the melting point of the solid polyethylene glycol (e.g. PEG3000) and dispersing the solid polyethylene glycol into the mixture;
(iii) adding powdered ingredients (such as abrasive cleaning agent and/or surfactant) to the mixture so obtained;
(iv) adding the thickening agent to the mixture so obtained, and
(v) cooling the mixture to form crystals of the solid polyethylene glycol(s).

Further optional ingredients (such as the fluoride sources and/or the agents for the remineralisation of teeth, as described above) may suitably be added after step (ii) and preferably at any stage between steps (ii) and (iv).

The invention is further illustrated with reference to the following, non-limiting Examples.

### EXAMPLES

Test formulations were prepared having the ingredients shown in Table 1 below. All ingredients are expressed by weight percent of the total formulation, and as level of active ingredient, unless otherwise specified.

**Table 1**

| Ingredient | Comparative Example | Example 1 | Example 2 |
|---|---|---|---|
| Glycerol (99.5% a.i.) | 54.889 | 54.1387 | 54.3887 |
| PEG 400 | 10.5 | 10.5 | 10.5 |
| PEG 3000 | 1.75 | 1.75 | 1.75 |
| Monosodium dihydrogen phosphate | 3.2 | 3.2 | 3.2 |
| Sodium monofluorophosphate | 1.11 | 1.11 | 1.11 |
| Sodium saccharin | 0.2 | 0.2 | 0.2 |
| Trisodium phosphate | 3.8 | 3.8 | 3.8 |
| D&C Red 30 (C.I.173360); 90% a.i. | 0.001 | 0.001 | 0.001 |
| Blue Covarine (C.I.74160); 40% a.i. | 0.0003 | 0.0003 | 0.0003 |
| Timiron®MP149 | 0.05 | 0.05 | 0.05 |
| Calcium silicate | 15 | 15 | 15 |
| Sorbosil®AC77 (ex PQ Corp.) | 6.000 | 6.000 | 6.000 |
| Sodium lauryl sulphate | 2.000 | 2.000 | 2.000 |
| Sorbosil®AC33 (ex PQ Corp.) | - | 1.000 | - |
| Sorbosil®AC43 (ex PQ Corp.) | - | - | 0.5000 |
| Synthalen®KP (ex 3V Sigma) | 0.300 | 0.300 | 0.300 |
| Flavour | 1.200 | 1.200 | 1.200 |

Samples of each of the formulations were evaluated for their visual appearance and also their ease of squeezability (extrudability) from a standard toothpaste packaging tube.

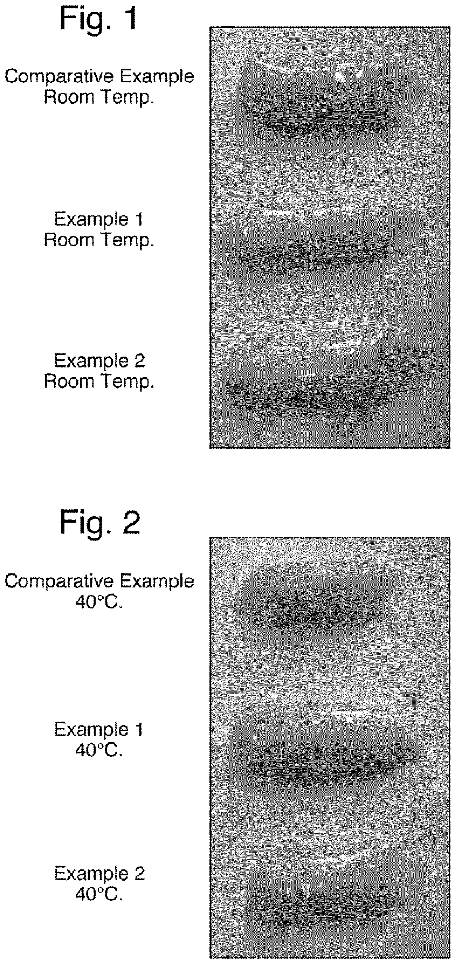

Figure 1 shows the results at room temperature. It can be seen that all three samples have a shiny, glossy and attractive appearance. All three samples were also observed to be easy to squeeze.

However, Figure 2 shows the results after the samples were heated to 40°C. It can be seen that visually, the sample of the Comparative Example has a significantly duller and cloudier appearance than either the sample of Example 1 or the sample of Example 2. Furthermore it was observed that the sample of the Comparative Example had a harder texture and was more difficult to squeeze, compared to either the sample of Example 1 or the sample of Example 2.

## Claims

1. A non-aqueous oral care composition with a liquid continuous phase comprising a thickening agent, a humectant, and a crystalline structuring agent, **characterised in that** the composition further comprises abrasive amorphous silica particles which are capable of acting as a booster to the cleaning ability of the composition and which have a weight mean particle, size (d₅₀) ranging from 3 to 6 microns; in which the thickening agent is a carboxyvinyl polymer having a molecular weight of at least 750,000 g/mol; in which the humectant is one or more organic polyols having 3 or more hydroxyl groups in the molecule and in which the crystalline structuring agent is a solid polyethylene glycol having a melting point ranging from 35 to 65°C

2. A composition according to claim 1, in which the carboxyvinyl polymer is a homopolymer of acrylic acid crosslinked with pentaerythritol triallylether.

3. A composition according to claim 1 or 2, In which the solid polyethylene glycol has the general formula H(OCH₂CH₂)ₙ OH, where n is the number of repeating oxyethylene units, and the average value of n ranges from 60 to 75.

4. A composition according to any one of claims 1 to 3, in which the liquid continuous phase further comprises one or more liquid polyethylene glycols having a melting point below 25°C.

5. A composition according to claim 4, in which the liquid polyethylene glycol has the general formula H(OCH₂CH₂)ₙ OH, where n is the number of repeating oxyethylene units, and the average value of n ranges from 4 to 12.

6. An oral care composition according to any one of claims 1 to 6, which is in the form of a dentifrice comprising other abrasive materials (In addition to the abrasive amorphous silica particles) in an amount of from 3 to 75% by weight based on the total weight of the dentifrice, and a surfactant in an amount of from 0.2 to 5% by weight based on the total weight of the dentifrice.

## Patentansprüche

1. Nicht-wässrige Mundpflegezusammensetzung mit einer flüssigen kontinuierlichen Phase, umfassend ein Verdickungsmittel, ein Feuchthaltemittel und ein kristallines Strukturierungsmittel, **dadurch gekennzeichnet, dass** die Zusammensetzung des Weiteren abrasive amorphe Siliziumdioxid-Partikel umfasst, die als ein Booster für die Reinigungsfähigkeit der Zusammensetzung dienen können und die eine gewichtsgemittelte Partikelgröße (d₅₀) in dem Bereich von 3 bis 6 Mikron aufweisen, worin das Verdickungsmittel ein Carboxylvinylpolymer mit einem Molekulargewicht von mindestens 750.000 g/Mol ist, worin das Feuchthaltemittel ein oder mehrere organische Polyole mit 3 oder mehr Hydroxylgruppen in dem Molekül darstellt und worin das kristalline Strukturierungsmittel ein festes Polyethylenglycol mit einem Schmelzpunkt von 35 bis 65°C ist.

2. Zusammensetzung nach Anspruch 1, worin das Carboxyvinylpolymer ein Homopolymer von Acrylsäure, vernetzt mit Pentaerythrittriallylether, ist.

3. Zusammensetzung nach Anspruch 1 oder 2, worin das feste Polyethylenglycol die allgemeine Formel H(OCH₂CH₂)ₙOH aufweist, worin n die Zahl der sich wiederholenden Oxyethylen-Einheiten darstellt und sich der durchschnittliche Wert von n von 60 bis 75 erstreckt.

4. Zusammensetzung nach irgendeinem der Ansprüche 1 bis 3, worin die flüssige kontinuierliche Phase des Weiteren ein oder mehrere flüssige Polyethylenglycole mit einem Schmelzpunkt unter 25°C umfasst.

5. Zusammensetzung nach Anspruch 4, worin das flüssige Polyethylenglycol die allgemeine Formel H(OCH₂CH₂)ₙOH aufweist, worin n die Zahl der sich wiederholenden Oxyethylen-Einheiten darstellt und sich der durchschnittliche Wert von n von 4 bis 12 erstreckt.

6. Mundpflegezusammensetzung nach irgendeinem der Ansprüche 1 bis 5, die in Form eines Zahnputzmittels vorliegt, das andere abrasive Materialien (zusätzlich zu den abrasiven amorphen Siliziumdioxid-Partikeln) in einer Menge von 3 bis 75 Gewichts-%, bezogen auf das Gesamtgewicht des Zahnputzmittels, und ein Tensid in einer Menge von 0,2 bis 5 Gewichts-%, bezogen auf das Gesamtgewicht des Zahnputzmittels, umfasst.

## Revendications

1. Composition de soin oral non aqueuse avec une phase continue liquide comprenant un agent épaississant, un humectant, et un agent structurant cristallin, **caractérisée en ce que** la composition comprend de plus des particules de silice amorphe abrasive qui peuvent agir comme un booster par rapport à l'aptitude de nettoyage de la composition et présente une taille moyenne de particule en masse (d₅₀) de 3 à 6 microns ; dans laquelle l'agent épaississant est un polymère de carboxyvinyle ayant une masse moléculaire d'au moins 750 000 g/mol ; dans laquelle l'humectant est un ou plusieurs polyols organiques ayant 3 groupes hydroxyle ou plus dans la molécule et dans laquelle l'agent structurant cristallin est un polyéthylène glycol solide ayant un point de fusion de 35 à 65°C.

2. Composition selon la revendication 1, dans laquelle le polymère de carboxyvinyle est un homopolymère d'acide acrylique réticulé avec un triallyléther de pentaérythritol.

3. Composition selon la revendication 1 ou 2, dans laquelle le polyéthylène glycol solide présente la formule générale H(OCH₂CH₂)ₙOH, où n est le nombre d'unités oxyéthylène répétitives, et la valeur moyenne de n est de 60 à 75.

4. Composition selon l'une quelconque des revendications 1 à 3, dans laquelle la phase continue liquide comprend de plus un ou plusieurs polyéthylène glycols liquides ayant un point de fusion inférieur à 25°C.

5. Composition selon la revendication 4, dans laquelle le polyéthylène glycol liquide présente la formule générale H(OCH₂CH₂)ₙOH, où n est le nombre d'unités oxyéthylène répétitives, et la valeur moyenne de n est de 4 à 12.

6. Composition de soin oral selon l'une quelconque des revendications 1 à 5, laquelle est dans la forme d'un dentifrice comprenant d'autres matières abrasives (en plus des particules de silice amorphe abrasive) dans une quantité de 3 à 75 % en masse rapportée à la masse totale du dentifrice, et un tensioactif dans une quantité de 0,2 à 5 % en masse rapportée à la masse totale du dentifrice.
